# FASCICULE DE BREVET EUROPEEN

(11) **EP 0 616 793 B1**
(45) Date de publication et mention de la délivrance du brevet: **03.01.1996**
(21) Numéro de dépôt: 93810602.8
(22) Date de dépôt: 24.08.1993
(51) Int. Cl.: A61B 17/22

(54) **Instrument médical pour l'élimination de dépôts formés sur les parois intérieures des artères ou des veines**
Medizinisches Instrument zum Entfernen von Ablagerungen auf den inneren Wänden von Arterien oder Venen
Medical instrument for removing deposits from the interior walls of arteries or veins

(30) Priorité: 25.03.1993 CH 914/93
(43) Date de publication de la demande: 28.09.1994
(73) Titulaire: FERROMEC S.A., CH-1302 Vufflens-la-Ville (CH)
(72) Inventeur: Kropf, Laurent, CH-1303 Penthaz (CH); Baumgartner, Hugues, CH-1074 Mollie-Margot (CH); Damone, Umberto, CH-1003 Lausanne (CH)
(74) Mandataire: Meylan, Robert Maurice

(56) Documents cités:
- EP-A- 0 431 743
- WO-A-89/09029
- WO-A-90/02523
- WO-A-91/08710
- DE-A- 1 935 856
- DE-A- 2 737 014
- FR-A- 1 585 065

## Description

La présente invention a pour objet un instrument médical pour l'élimination de dépôts formés sur les parois intérieures des artères ou des veines, comprenant un tube extérieur fixé à l'extrémité d'un tube ou gaine flexible destiné à être introduit dans l'artère ou la veine, un tube intérieur déplaçable longitudinalement dans le tube extérieur, ce tube intérieur étant muni, à son extrémité distale, d'une tête convexe présentant un passage axial prolongeant le tube intérieur, des moyens de coupe et de raclage extensibles et des moyens pour assurer l'expansion et la rétraction des moyens de coupe et de raclage sous l'effet du déplacement longitudinal relatif des deux tubes.

Dans le brevet US 5 074 871 est décrit un instrument de ce type, dans lequel les moyens de coupe et de raclage sont constitués par des bras flexibles dont les extrémités sont articulées d'une part sur la tête convexe solidaire du tube intérieur et d'autre part sur l'extrémité du tube extérieur. Lorsque la tête convexe est rapprochée de l'extrémité du tube extérieur, les bras flexibles fléchissent par flambage de telle manière qu'on obtient une expansion du dispositif de coupe. Dans la partie médiane de certains des bras est en outre découpée une languette agressive constituant le moyen de coupe proprement dit. La rétraction des bras flexibles est assurée par leur élasticité propre ainsi que par le déplacement en sens opposé de la tête. Cette tête présente un trou axial permettant le passage d'un fil de guidage ou d'un cathéter pour le gonflement d'un ballonnet. Si l'on considère que selon les normes fixées aujourd'hui en chirurgie, le diamètre de l'introducteur ne doit pas dépasser 3 mm, de telle sorte que l'instrument doit présenter un diamètre sensiblement inférieur à 3mm (pour permettre d'étancher le sang), on peut se rendre compte des faibles dimensions des axes d'articulation des lames flexibles et partant de la fragilité de ces articulations. Non seulement les lames flexibles et leurs attaches à la tête et au tube extérieur sont très fragiles, mais le problème de l'élimination des matériaux arrachés à la paroi du vaisseau sanguin n'a pas été résolue. Il est prévu, dans ce document, de retirer les matériaux arrachés au moyen d'un cathéter muni d'un ballonnet ou d'une membrane entourant la tête de coupe constituée des bras flexibles. Dans les deux cas, l'élimination du matériau arraché n'est pas du tout assurée, mais au contraire, une importante quantité de matériau risque d'être entraînée dans le flux sanguin.

Du brevet US 4 794 928, on connait également un instrument d'angioplastie comprenant un cathéter et des moyens de coupe expansibles constitués de lames. Ces lames sont soit articulées sur un support et commandées par des cames, ou constituées de bras élastiques écartés par le gonflement d'un ballonnet. Aucun moyen n'est prévu pour retenir et éliminer les matériaux arrachés à la paroi du vaisseau sanguin.

Le brevet DE 38 30 704 décrit un instrument dans lequel des lames en forme de coquille sont montées sur un support en matière élastiquement déformable dont la déformation par un fil de traction assure l'expansion des coquilles.

Il a également été proposé de monter des couteaux rigides à l'extrémité d'un cathéter et de commander l'écartement de ces couteaux par un fil agissant sur des biellettes articulées en un point intermédiaire des couteaux. Un tel mécanisme est non seulement fragile, vues les faibles dimensions dans lesquelles il doit être exécuté, mais il est en outre mécaniquement défavorable compte tenu du rapport des forces en présence. En particulier, lorsque les couteaux, écartés, ont raclé une certaine quantité de matériau, il n'est pratiquement plus possible de refermer les couteaux. Le problème d'élimination des matériaux arrachés à la paroi du vaisseau sanguin n'a en outre pas été résolu. De plus, il n'est pratiquement pas possible d'avoir un tube intérieur pour l'injection d'un liquide.

La présente invention a pour but de réaliser un instrument obviant aux inconvénients des instruments connus. Plus précisément, l'invention a pour but de réaliser un instrument très robuste ne comportant pas d'articulation, ni autre pièce fragile et dans lequel la rétraction des moyens de coupe et de raclage est assurée en toute circonstance et qui permet d'éliminer, c'est-à-dire de retirer des vaisseaux sanguins la totalité des matériaux arrachés à la paroi du vaisseau sanguin.
l'instrument médical selon l'invention est dèfini à la revendication 1.

Cette conception permet de réaliser une construction particulièrement robuste sans articulation fragile, d'un fonctionnement fiable et qui ne comporte que des pièces ridiges dont la section peut assurer leur parfaite solidité. Cette solidité permet en particulier d'assurer la rétraction des couteaux par le manteau, même en présence d'une accumulation, entre les couteaux, de matériaux arrachés à la paroi du vaisseau sanguin.

D'autre part, les matériaux arrachés peuvent être entièrement retenus à l'intérieur du manteau cylindrique, celui-ci étant fermé, en position rétractée, par l'extrémité du tube extérieur.

Le tube intérieur est utilisé, de manière conventionnelle, pour le passage d'un fil de guidage du cathéter lors de son introduction dans l'artère ou la veine à traiter. Ce tube intérieur peut également être utilisé pour l'injection d'un liquide de traitement ou de contraste. Le nombre de couteaux peut bien entendu être supérieur à deux, par exemple égal à trois ou quatre.

Le dessin annexé représente, à titre d'exemple, une forme d'exécution de l'invention.

La figure 1 est une vue en coupe axiale de l'instrument en position rétractée des couteaux.

La figure 2 est une vue analogue en position expansée des couteaux.

L'instrument représenté comprend un tube extérieur 1 rigide, destiné à être fixé à l'extrémité d'une gaine 2 par son extrémité proximale 4 de diamètres intérieur et extérieur plus grand que les diamètres correspondants du reste du tube 1, et un tube intérieur 3 souple de diamètre extérieur sensiblement égal au diamètre intérieur du tube extérieur 1 et traversant la gaine 2 sur toute sa longueur ainsi que le tube extérieur 1. L'extrémité distale du tube intérieur 3 est munie d'une tête 5 convexe, présentant plus particulièrement une calotte émisphérique, traversée par un passage axial 6 prolongeant le tube intérieur 3. A cette tête 5, est fixé un manteau cylindrique 7 entourant le tube extérieur 1 et s'étendant sur presque toute la longueur de celui-ci. Ce manteau 7 est guidé axialement par un flasque cylindrique 8 formé à l'extrémité distale du tube extérieur 1, flasque dont le diamètre est sensiblement égal au diamètre intérieur du manteau 7, dont le diamètre extérieur est égal au diamètre de la tête 5 et au diamètre de la partie proximale 4 du tube extérieur 1. Le manteau 7 a un diamètre extérieur de 2,7 mm.

A l'intérieur du manteau 7, entre ce manteau et le tube extérieur 1, sont montées, symétriquement relativement à l'axe 9 de l'instrument, deux couteaux 10 et 11 en forme de selle de cheval allongée, c'est-à-dire de corps dont les faces sont définies, au moins approximativement, par deux hyperboloïdes de révolution coaxiaux. Chacun des couteaux présente donc, dans la direction de l'axe, une face extérieure concave 12 et une face intérieure convexe 13. Les couteaux 10 et 11 sont en appui contre le tube extérieur 1 par leur face convexe 13. Sur le tube extérieur 1, à proximité de l'extrémité 8, est fixée une bague arrondie 14 de forme semi-torique retenant axialement les couteaux 10 et 11 et sur laquelle les couteaux 10 et 11 peuvent pivoter, les couteaux présentant à cet effet une gorge de forme adaptée à celle de la bague.

Le manteau 7 présente en outre sur sa face intérieure un bossage annulaire 15 dont la fonction de came sera exposée ci-après dans la description du fonctionnement de l'instrument.

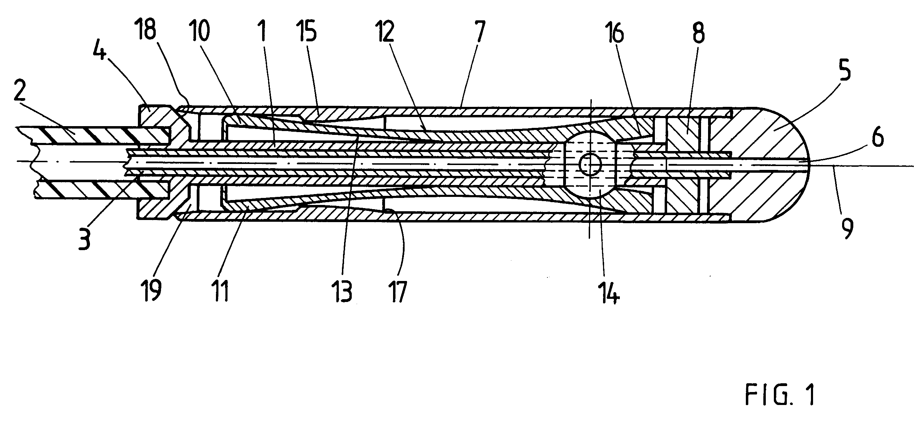

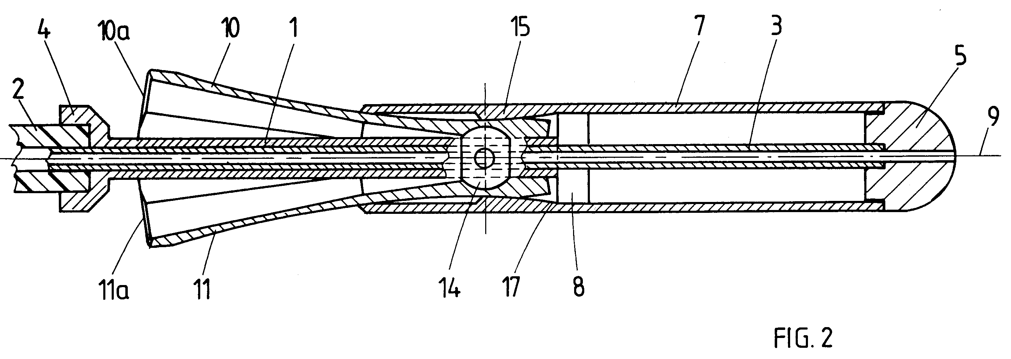

Partant de la position rétractée ou fermée représentée à la figure 1, si l'opérateur désire écarter les couteaux 10 et 11 pour opérer un raclage de la paroi interne d'un vaisseau sanguin, il opère, au moyen d'une poignée adéquate, le déplacement relatif longitudinal des tubes 1 et 3, soit par traction sur le tube extérieur 1, soit par poussée du tube intérieur 3. Ces deux actions peuvent être conjuguées. La poignée d'actionnement peut être par exemple du type décrit dans le brevet US 5 074 871 ou d'une autre forme connue. Lors de ce déplacement, le bossage 15 vient buter sur les couteaux 10 et 11 ce qui a pour effet de faire basculer les couteaux sur le tube extérieur 1. Ce basculement est limité, d'une part, par la partie terminale 16 de la face intérieure des couteaux qui vient buter contre le tube extérieur 1 et, d'autre part, par un épaulement 17, formé à l'extrémité du bossage 15, contre lequel vient buter le flasque cylindrique 8. Les couteaux sont ainsi maintenus dans la position ouverte représentée à la figure 2 par blocage entre le bossage 15 et le tube extérieur 1. Les couteaux 10 et 11 présentent un tranchant 10a, respectivement 11a permettant d'attaquer le dépôt de la paroi du vaisseau sanguin par une traction sur la gaine 2. Le matériau arraché s'accumule entre les couteaux et le tube extérieur 1.

L'opération de raclage terminée, on exerce une traction sur le tube intérieur 3, ce qui a pour effet de ramener le manteau cylindrique 7 en direction de l'extrémité 4 du tube extérieur 1. Lors de ce déplacement, le bossage 15 libère les couteaux 10 et 11 pour permettre, simultanément, à l'extrémité du manteau 7 d'agir à son tour comme une came sur les couteaux 10 et 11 pour les faire basculer dans l'autre sens, c'est-à-dire les refermer. Le manteau 7 étant une enveloppe cylindrique fermée, la force radiale qu'il peut exercer sur les couteaux 10 et 11 est considérable et les matériaux accumulés entre les couteaux 10 et 11 n'empêchent pas la fermeture de ceux-ci.

Lorsque le manteau 7 arrive en bout de course, son extrémité vient buter contre un chanfrein 18 de l'extrémité 4 du tube 1 qui vient fermer totalement l'enceinte constituée par le manteau cylindrique 7 et le tube extérieur 1. Les matériaux arrachés au vaisseau et accumulés dans le manteau 7 peuvent ainsi être retirés dans leur totalité du vaisseau sanguin. En raison de la fermeture des couteaux, le matériau s'accumule essentiellement dans une chambre annulaire 19 formée devant la partie 4 du tube 1.

## Revendications

1. Instrument médical, pour l'élimination de dépôts formés sur les parois intérieures des artières ou des veines, comprenant un tube extérieur (1) fixé à l'extremité d'une gaine ou tube flexible (2) destiné à être introduit dans l'artère ou la veine, un tube intérieur (3) déplaçable longitudinalement dans le tube extérieur, ce tube intérieur étant muni, à son extrémité distale d'une tête convexe (5) présentant un passage axial prolongeant le tube intérieur, des moyens de coupe et de raclage expansibles (10, 11) et des moyens (7, 15) pour assurer l'expansion et la rétraction des moyens de coupe et de raclage sous l'effet du déplacement longitudinal relatif des deux tubes, caractérisé par le fait que la tête convexe (5) est munie d'un manteau cylindrique (7) entourant le tube extérieur (1) et de diamètre extérieur égal au diamètre de la tête (5), que le tube extérieur (1) présente, à son extrémité proximale, une partie (4) de diamètre au moins approximativement égale au diamètre extérieur du manteau cylindrique (7) et à son extrémité distale, des moyens de guidage (8) du manteau, que les moyens de coupe et de raclage sont constitués d'au moins deux couteaux rigides (10, 11) substantiellement en forme d'hyperboloïdes de révolution s'étendant longitudinalement le long et autour du tube extérieur, à l'intérieur dudit manteau et s'appuyant par leur face convexe sur le tube extérieur, les extrémités des couteaux étant au moins approximativement en contact avec la paroi du manteau en position rétractée et que les moyens pour assurer l'expansion des couteaux sont constitués d'un bossage intérieur (15) du manteau ayant la fonction d'une came dont le déplacement longitudinal fait basculer les couteaux sur le tube extérieur (1), la rétraction des couteaux étant assurée par l'extrémité du manteau (7).

2. Instrument selon la revendication 1, caractérisé par le fait qu'en position rétractée des couteaux, l'extrémité du manteau (7) vient en contact avec l'extrémité proximale (4) du tube extérieur de manière à fermer l'espace délimité par le manteau.

3. Instrument selon la revendication 1 ou 2, caractérisé par le fait que le tube extérieur (1) est muni d'un moyen (14) de retenue axiale des couteaux.

## Claims

1. Medical instrument for the removal of deposits formed on the inner walls of the arteries or veins, comprising an outer tube (1) fixed to the end of a flexible sheath or tube (2) intended to be introduced into the artery or the vein, an inner tube (3) displaceable longitudinally in the outer tube, this inner tube being equipped, at its distal end, with a convex head (5) exhibiting an axial passage continuing the inner tube, expandable cutting and scraping means (10, 11) and means (7, 15) for ensuring the expansion and retraction of the cutting and scraping means under the effect of the relative longitudinal displacement of the two tubes, characterized by the fact that the convex head (5) is equipped with a cylindrical casing (7) surrounding the outer tube (1) and of an external diameter equal to the diameter of the head (5), that the outer tube (1) has, at its proximal end, a part (4) having a diameter at least approximately equal to the external diameter of the cylindrical casing (7), and at its distal end, means (8) for guiding the casing, that the cutting and scraping means are formed by at least two rigid blades (10, 11) substantially in the shape of a hyperboloid of revolution extending longitudinally along and around the outer tube, inside said casing, and bearing via their convex face on the outer tube, the ends of the blades being at least approximately in contact with the wall of the casing in the retracted position, and that the means for ensuring the expansion of the blades are formed by an inner boss (15) on the casing, having the function of a cam, the longitudinal displacement of which causes the blades to swivel on the outer tube (1), the retraction of the blades being effected by the end of the casing (7).

2. Instrument according to claim 1, characterized by the fact that, in the retracted position of the blades, the end of the casing (7) comes into contact with the proximal end (4) of the outer tube, in such a way as to close the space delimited by the casing.

3. Instrument according to claim 1 or 2, characterized by the fact that the outer tube (1) is equipped with a means (14) for axial retention of the blades.

## Patentansprüche

1. Medizinisches Instrument zum Entfernen von Ablagerungen auf den inneren Wänden von Arterien oder Venen, welches ein äusseres Rohr (1) welches am Ende einer flexiblen Umhüllung oder eines flexiblen Rohrs (2) befestigt ist, die oder das dazu vorgesehen ist, in die Arterie oder Vene eingeführt zu werden, ein inneres Rohr (3), das in Längsrichtung in dem äusseren Rohr (1) verschiebbar ist und an seinem von der Instrumentenbasis entferntesten Ende einen konvexen Kopf (5) mit einem axialen, das innere Rohr verlängernden Durchgang aufweist, dehnbare Schneid- und Schabmittel (10, 11) und Mittel (7, 15) für die Ausdehnung und den Rückzug der Schneid- und Schabmittel unter der Wirkung der Bewegung in Längsrichtung der zwei Rohre relativ zueinander aufweist, dadurch gekennzeichnet, dass der konvexe Kopf (5) mit einem zylindrischen Mantel (7) versehen ist, welcher das äussere Rohr (1) umhüllt und dessen äusserer Durchmesser gleich dem Durchmesser des Kopfes (5) ist, dass das äussere Rohr (1) an seinem von der Instrumentenbasis nächstliegenden Ende ein Teil (4) von mindestens etwa dem gleichen Durchmesser wie der äussere Durchmesser des zylindrischen Mantels (7) und an seinem von der Instrumentenbasis entferntesten Ende Mittel (8) zur Führung des Mantels aufweist, dass die Schneid- und Schabmittel mindestens aus zwei steifen Messer (10, 11) bestehen, welche im wesentlichen die Form von Umdrehungshyperboloide haben, die sich im genannten Mantel in Längsrichtung längs des äusseren Rohrs und um dieses erstrecken, und sich mit ihrer konvexen Seite auf das äussere Rohr stützen, wobei die Enden der Messer wenigstens annähernd in zurückgezogener Stellung mit der Mantelwand in Kontakt stehen und dass die Mittel zur Ausdehnung der Messer aus einem inneren Vorsprung (15) des Mantels bestehen, wobei dieser Vorsprung die Funktion eines Nockens hat, dessen Bewegung in Längsrichtung die Messer auf das äussere Rohr (1) kippen lässt, wobei der Rückzug der Messer durch das Ende des Mantels (7) gewährleistet wird.

2. Instrument nach Anspruch 1, dadurch gekennzeichnet, dass das Ende des Mantels (7) in zurückgezogener Stellung der Messer in Kontakt mit dem Ende (4) des äusseren Rohrs kommt, das von der Instrumentenbasis am weitesten entfernt ist, um den durch den Mantel begrenzten Raum zu schliessen.

3. Instrument nach Anspruch 1 oder 2, dadurch gekennzeichnet, dass das äussere Rohr (1) mit einem Mittel (14) zur axialen Halterung der Messer versehen ist.
